# EUROPEAN PATENT APPLICATION

(11) **EP 4 560 016 A1**
(43) Date of publication of application: **28.05.2025**
(21) Application number: 23307033.3
(22) Date of filing: 22.11.2023
(51) Int. Cl.: C12N 9/22, C12N 15/82

(54) **METHOD OF PLANT GENOME EDITING**

(71) Applicant: Plantik Biosciences, 45006 Orleans (FR)
(72) Inventor: NAYAK, Aditya, ORLEANS (FR)
(74) Representative: Cabinet Beau de Loménie

(57) **Abstract**

The invention, concerning the field of plant genome editing, relates to a method of transforming pollen grains, a method of genome editing pollen grains, and a method of obtaining a genome edited homozygous plant.

## Description

### FIELD OF THE INVENTION

The invention, concerning the field of plant genome editing, relates to a method of transforming pollen grains, a method of genome editing pollen grains, and a method of obtaining a genome edited homozygous plant.

### BACKGROUND OF THE INVENTION

Plant genetic manipulation is an ancestral practice starting with the unconscious domestication of actual major crops. As an example, it has been reported that maize domestication started in tropical Balsas river valley in Mexico 7000 to 10000 years ago from its wild precursor, teosinte. To obtain the actual maize varieties, former farmers selected seed coming from plants with desirable phenotypic traits and discard the one with undesirable phenotype, leading to considerable reduction of genetic diversity by increasing the proportion of a desirable allele. This process, based on natural selection, is then the ancestry of plant breeding techniques. Despite the fact that most of the actual known crops were obtained following this process, pre-existing traits come to its limit with the time scale of adaptation (mutational events occurring at low frequencies, i.e., 10⁻⁵ to 10⁻⁸ per locus.

To counteract, breeders have investigated breeding method based on pedigree, consisting in crossing parents with desirable traits to generate a segregation population which are conducted through generations of self- pollination and selection, until a set of derived lines that combines the good characteristics of both parents is obtained. However, as it is not possible to predict the proportion and recombination between the genome of both parents, molecular markers have been developed in order to monitor the transmission of the desired traits in the progeny. Even if these methods have largely proved their worth through the selection of quantitative traits of interest (yield, resistance to disease...), they present the disadvantage of being extremely expensive, time consuming insofar as the creation of a variety is estimated at about 10 years. Alternative methods aiming at increasing genetic diversity through induced mutation by chemical or physical agent have considerably helped to accelerate breeding programs. Billions of mutations are randomly induced and the mutant population is then screened for desirable traits and backcrossed to wild genotype to clean the genome from non-desirable mutation and preserve the mutated portion.

Also, considerable advances in molecular biology made it possible for humans to modify plant genomes. One of the most relevant examples is the creation of the golden rice varieties by Ingo Potrykus and Peter Beyer in 1990s which contains a bioengineered multigene biochemical pathway to produced beta-carotene, a precursor of vitamin C. Genetic engineering techniques were also used on the cotton plant to insert genes that encode a protein toxic to certain caterpillar pests common to cotton and other crops. Today, 125,000 ha of "Bt cotton" were planted by Burkina Faso producers, marking it the largest-ever introduction of biotechnology on the African continent.

During the past ten years, molecular tools have been developed for precise genome editing. The well-known type II CRISPR/Cas system, such as CRISPR/Cas9 system, has been successfully used in plants for increasing yield, increasing quality, diseases and herbicide resistance. The CRISPR/Cas machinery comprised three elements: the CRISP RNA(crRNA), the trans-activating crRNA (tracrRNA) and the Cas protein. Cas9 has the DNA cleavage domains which break the double-stranded DNA site primarily located 3 bp upstream of protospacer adjacent motif (PAM) sequences in the target DNA. As a result, DNA is repaired *in vivo* using error-prone nonhomologous end-joining (NHEJ) or homology-directed repair (HDR) inducing insertion, deletion, sequence replacement and/or nucleotide substitutions. Genetic modification of plants is conditioned by the delivery of the molecular machinery necessary for genome edition. Genetic engineering in plants can be classically summarised in four main steps (i) Isolating the DNA fragment of interest, (ii) Cloning it into a plasmid, (iii) Delivery of the given DNA in plant cells and integration in the genome, and (iv) Regeneration of an entire plant from the transformed cell.

Indirect methods of DNA delivery, such as Agrobacterium mediated transformation, and direct methods of DNA delivery, such as biolistic DNA delivery, are the main method for DNA delivery.

Agrobacterium mediated transformations have been widely used, mostly on dicotyledons, to deliver recombinant nucleic acid inside plant tissues. Natural virulent strains are known to infect plants by transferring a plasmid, namely Ti plasmid, a tumour inducing agent inside the plant cell. Thanks to the vir genes coded by the TI plasmid, a portion of this later, the TDNA, is then transferred inside the infected cells. The TDNA is flanked by a right and left border allowing the fragment to be inserted steadily in the plant genome. The natural system has been modified in order to transfer and integrate any DNA fragment of interest. Once integrated, the T-DNA behaves like a normal plant gene, steadily maintained and transferred to the progeny. Infection through agrobacterium consists in putting the transgenic bacteria solution in contact with the chosen tissue, which differs in monocot and dicot. In dicotyledon, cotyledons, internodal segment, hypocotyl, protoplast and embryonic calli have been used for successful transformation and regeneration processes. Monocotyledon Agrobacterium mediated transformation has been a big challenge until recently but have been successfully used on pre cultured immature embryo, Embryogenic callus, freshly isolated immature embryo, suspension culture, Immature embryo, meristem corn slices and pollen culture. Other alternative methods of infection such as germ-line transformation via vacuum infiltration, seed transformation, in planta transformation, and floral-dip, allow the avoidance of tissue culture and regeneration but their efficiency is very limited to some plants such as *Arabidopsis* and some *Brassicaceae.* More generally, plant species largely differed in their susceptibility to Agrobacterium infection and even within a species, the ecotypes or varieties may not react the same way. Agrobacterium mediated transformation presents many advantages like minimal equipment, relatively cheap, low transgene copy number, minimal DNA rearrangement and somaclonal mutation, and relatively high stability of the transgene.

On the other hand, in the case of biolistic DNA delivery, DNA coating particles made of gold or tungsten are shot into the target plant tissue under high pressure allowing them to penetrate through the cell wall and plasma membrane and deliver the foreign DNA directly in the nucleus. The DNA will then elute from the particles and integrate directly in the plant chromosomes. This method has been successfully used in species recalcitrant to Agrobacterium transformation, such as rice, wheat (and maize. Major concern about the biolistic method resides in its random character, multi-copy transgene integration, resulting in irregular compositions of inverted repeats or transgene rearrangements. These can lead to transgene silencing or aberrant expression. Electroporation can also be used as a method for membrane permeabilization thanks to an induced voltage leading to an imbalanced electrical potential in the plasma membrane. The effect of this electrical imbalance is reversible in such a way the DNA can be introduced into the cells without changing the cellular functions or the membrane integrity. Because of the presence of a cell wall surrounding plant cells, this method has mostly been used for protoplast transformation. Other methods aiming at permeabilizing the membrane such as PEG mediated have been successfully used, especially in cereal for transient or permanent protoplast transformation. The two most critical requisites are the ability to isolate and culture protoplasts in large numbers, and the development of an efficient protocol from plant regeneration. Mesophyll protoplast of dicotyledon species showed a quite good efficiency of transformation and regeneration. However, to date, there is no clear evidence of induced cell division in mesophyll protoplast from monocot. In contrast, protoplasts isolated from embryogenic suspension cultures could be induced to divide, making protoplast transformation a very challenging method for plant stable transformation, especially in cereals.

However, almost all the previously cited methods imply *in vitro* culture and regeneration of the plant. Despite considerable progress, plant regeneration remains one of the biggest challenges of plant transformation. In vitro plant regeneration can be performed via somatic embryogenesis or organogenesis. Organogenesis is the process by which first a new organ and then the entire plant is regenerated, in response to various hormonal stimuli, from other parts of the organs, tissues, or protoplast. It has been shown that high auxin/cytokinin ratio in the medium induces organogenic callus from an explant and, subsequently, a high cytokinin/auxin ratio triggers shoot formation. In somatic embryogenesis, first, a structural cell similar to zygotic embryos is formed among other parameters, thanks to high concentrations of auxin, and then the entire plant is regenerated. Plant regeneration is highly dependent on the plant species, the genotype and the explant but also physico chemical parameters such as the culture medium, phytohormones, genotype, carbohydrate, and gelling agent, as well as some other agents such as light regime, temperature, humidity, etc. Also, in addition to the stress reaction leading to the production of ROS species, in vitro tissue culture cannot be dissociated from somaclonal variation resulting in mutations and chromosome rearrangement during the process of dedifferentiation/redifferentiation.

To overcome the regeneration steps, germ cell transformation and more precisely pollen transformation is probably the most promising alternative. In its original form, pollen tube mediated transformation implies removing a part of the stigma just after pollination and pollen tube germination and applying the solution of foreign linear DNA directly on the resulting style. The foreign DNA is conducted directly to the ovary through the pollen tube and is integrated within the undivided but fertilized recipient zygote. A variation of the method, called ovary dip transformation, aims at reducing the distance to travel for the transgenes and consists in the application of DNA solution containing short linear DNA fragments directly to the wound sites created at the ovaries. However, the transformation efficiency of this method as well as its reproducibility has been proven to be very low and thus requires a big effort in selecting the transformed lines among a large number of progeny. In addition, germinating pollen grain releases large amounts of nuclease that digest the foreign DNA in less than 5 to 10 minutes and then largely participate in reducing the efficiency of the method.

It has then been suggested to use the pollen grain itself as a vector by inducing DNA uptake. Different methods of pollen transformation have been reported:
- Incubation of dry pollen with an aqueous solution containing the foreign DNA, the idea was that the foreign DNA will be absorbed by imbibition (Hess et al., 1980). However, the effectiveness of the method has not been confirmed by more recent studies (Stöger et al., 1991).
- PEG transformation with plasmid DNA harboring the kanamycin resistance (Negrutiu et al., 1986)
- Agrobacterium mediated transformation coupled or not with coated particle bombardments (Abdollahi et al., 2009, Brew-Appiah et al., 2013).
- Particle bombardment was used to obtain chimeric genes containing pollen specific promoters that were transiently expressed in tobacco pollen (Twell et al., 1989, 1991).
- Electroporation especially in tobacco (Matthews et al., 1990), in addition to pretreatment with EDTA or MgSO4 to reduce the nuclease activity also showed promising results in maize (Van Wert et al., 1992). However, the transformation efficiency from 1 to 5% remains very low (Abdul Baki et al, 1990).
- Ultrasonication of pollen grains to remove the operculum covering the pollen germination pore, making the pollen grains become more competent to receive foreign DNA (Yang et al., 2017).

Methods for pollen-mediated gene transformations of plants have also been described in US 5,049,500 and US 5,629, 183, in which the method involves introducing a foreign DNA into the pollen, e.g. by electroporation. However, this results in a foreign DNA in the final plant, which is undesirable for many applications.

WO2019113000 also discloses a method involving transformation of pollen grains using Cas9 and a gRNA and a cell penetrating peptide (CPP) for transforming pollen grains. However, cell penetrating peptides have a low effectiveness for pollen because the outer cell wall is made of sporopollenin which is a highly branched large carbon polymer that does not guarantee transformation of intact pollen grains. Once the pollen tube is germinated such peptides could help deliver cas9 and gRNA but then the chance of fertilization is poor due to the already germinating pollen grains.

Also, the recent development of nanotechnologies offers promising alternatives for DNA delivery into pollen grains. To date, different types of nanoparticles have already been used in plant sciences for stable transformation. Magnetic nanoparticles have been successfully used in maize (Wang et al., 2022) and cotton (Zhao et al., 2017) where polyethyleneimine coated Fe3O4 magnetic nanoparticles (MNP) are used as carriers for foreign DNA. After its formation, the complex is incorporated in the pollen grain, through the germination operculum thanks to a magnetic field. The transformed pollen grains are then used for artificial pollination. Despite its good efficiency, pollen magnetofection leads to more than one integration site of the transgene in the genome, requiring extra generation of self-pollination. In addition, the success rate of the transgenic events was affected by multiple factors, including pollen viability, pollination timing, pollination technique and environment conditions that need to be optimized depending on the genotype (Wang et al., 2022). Single-walled carbon nanotubes (SWNT) have also been used as an effective DNA carrier for mesophyll cell transformation and transient expression of GFP protein (S. Demirer et al., 2019, Kwak et al., 2019) and stable transformation pollen grain (Salim Lew et al., 2020). In this later, cationic polymer groups (polyethylenimine, chitosan or imidazolium) are attached to the sidewall of carboxylic acid-functionalized SWNTs via amide conjugation to allow the formation of DNA/SWNT complexes, with a net positive charge, thanks to electrostatic attraction. On the contrary to MNP, SWNT exploited the Lipid Exchange Envelope Penetration (LEEP) to penetrate the pollen grain, presenting the advantage of being independent of the entrance pathway through the operculum (Wong et al., 2016, Salim Lew et al., 2020). In the same way, Serag et al., 2011 showed that coated carbon nanotubes with cellulase immobilised on their side walls could penetrate the cell wall and transport DNA intracellularly through cellulase-induced nanoholes. This nano strategy shows excellent transformation efficiency, good biocompatibility, as well as the potential for plant regeneration. However, the gene delivery efficiency in the process of transformation is largely conditioned by the stability of nanoparticle colloids depending on the ionic strength of the buffer and/or its osmotic concentration. Therefore, many plant cell culture buffers are potentially incompatible with nanoparticles (Lv et al.2020).

Hemp (*Cannabis sativa*) has been one of the most important crops of mankind for well over 10,000 years. It is a crucial source of food, fibre, and medical comfort with more than 50,000 different known uses. However, in the last century, hemp has been stigmatised and prohibited, leading to its huge lack of scientific knowledge and development. As a result, *Cannabis sativa* counts less than 5% available varieties today compared to other major crops. As a result, methods to study gene function and their subsequent regulation are urgently needed. Despite its economic potential, hemp includes all the difficulties encountered in in vitro culture. Very few successful protocols of stable transformation and regeneration have been published in the past years. The first successful protocol was published in 2010. Hemps transformation is made from hypocotyls or immature embryos through agrobacterium mediated transformation. However, the regeneration efficiency of 5% is still very low. By overexpressing cannabis developmental regulator chimera in the embryo hypocotyls of immature grains, the shoot regeneration efficiency was substantially increased to 7%.

In order to overcome the difficulties related to the methods of the prior art, in particular plant transformation and regeneration, and to increase the transformation rate, the applicant proposes an efficient and easy-to-implement method of transforming pollen grains. The method comprises a step of incubating a ribonucleoprotein (RNP) complex and a sonicated pollen grain into a microdroplet, thereby obtaining a transformed pollen grain. The transformed pollen grain is then used for fertilizing (i.e. pollinating) a plant ovule thereby obtaining a gene edited homozygous plant. The transformed pollen grain is directly used for ovule fertilization and, thanks to RNP complex stability, the ovule genome is also edited during fertilization. The resulting embryo is thus homozygous for the desired gene edition (e.g. the desired mutation) and the resulting seed can be used to obtain a complete, homozygous plant, without any remaining RNP complex, in one generation.

### SUMMARY OF THE INVENTION

As mentioned above, the inventors interestingly propose a new approach for transforming pollen grains and for obtaining a gene edited homozygous plant in one generation.

Thus, a first object of the invention concerns a method of transforming pollen grains, the method comprising the steps of:
(a) providing pollen grains having at least one pore,
(b) providing a genome editing composition, the composition comprising a ribonucleoprotein (RNP) complex comprising (i) a RNA-guided endonuclease or a nucleic acid comprising a nucleotide sequence encoding the RNA-guided endonuclease and (ii) a guide RNA (gRNA) or a nucleic acid comprising a nucleotide sequence encoding the gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby obtaining transformed pollen grains.

A second object of the invention concerns a method of gene editing pollen grains, the method comprising the steps of:
(a) providing pollen grains having at least one pore,
(b) providing a genome editing composition, the composition comprising a ribonucleoprotein (RNP) complex comprising (i) a RNA-guided endonuclease or a nucleic acid comprising a nucleotide sequence encoding the RNA-guided endonuclease and (ii) a guide RNA (gRNA) or a nucleic acid comprising a nucleotide sequence encoding the gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby generating gene edited pollen grains.

A third object of the invention concerns a method of obtaining a gene edited homozygous plant, the method comprising the step of pollinating a plant ovule with a pollen grain obtained by the method of transforming pollen grains according to the invention or a method of gene editing pollen grains according to the invention, thereby obtaining a gene edited homozygous plant.

### DETAILED DESCRIPTION OF THE INVENTION

### Definitions

Unless otherwise defined, all technical and scientific terms used herein have the same meaning as commonly understood by one of ordinary skill in the art of molecular biology, medicinal chemistry, and/or organic chemistry. Although methods and materials similar or equivalent to those described herein can be used in the practice or testing of the present disclosure, suitable methods and materials are described herein.

The term "pollen grain" refers to the male gametophyte that contains the male gamete produced by flowers of seed plants. A pollen grain has a double wall: the vegetative and generative cells are surrounded by a thin delicate wall of unaltered cellulose called the endospore or intine, and a tough resistant outer cuticularized wall composed largely of sporopollenin called the exospore or exine. The first characteristics to be considered when identifying pollen grains are the apertures. An aperture is a thin or missing part of the exine, which is independent of the patterning of the exine. Two different types of apertures can be distinguished: pores and fissures (colpi, singular: colpus). Colpi and pores are major features in the identification of classes of pollen. Colpi are more primitive, they are elongated with pointed ends. Pores are usually isodiametric. Pores can also be slightly elongated but, in contrast to colpi, they have rounded ends. In some pollen grains, the exine around the apertures is either thicker or thinner. In pores this border is termed annulus (typical in grass pollen) and in colpi margo (e.g. in Hedera helix). Pollen grains with pores are porate and those with colpi are colpate. If both pore and colpus are combined in the same aperture, the pollen grain is colporate. Pollen grains can be divided into groups according to the number, position and type of apertures. This classification is simple and consistent. The number of apertures is indicated by the prefixes mono-, di-, tri- tetra-, penta-, hexa- and poly- with the above terms porate, colpate or colporate. Usually three pores and/or colpi are present that are regularly spaced around either the edge or the equator of the pollen grain, depending on whether the pollen grain is seen from the polar or equatorial view. If more than three apertures are present, they can either be regularly spaced around the edge, or equator respectively, (zonoporate/zonocolpate), or over the entire surface (pantoporate/ pantocolpate).

Pollen grains used in the present invention have at least one pore. Therefore, the term "pollen grains having at least one pore" refers to a porate or a colparate pollen grains. The pollen grains having at least one pore may be (i) a porate, such as a monoporate, a diporate, a triporate, a zonoporate, a pantoporate or a fenestrate, or (ii) a colporate, such as a tricolporate, a zonocolporate or a pantocolporate.

The term "transforming" refers to the introduction of a nucleic acid (e.g., DNA or RNA) or a protein into pollen grains. In the context of the invention, "transforming" refers to introducing a RNP complex into pollen grains, preferably as to allow gene editing the pollen grains genome.

The terms "polynucleotide" and "nucleic acid," used interchangeably herein, refer to a polymeric form of nucleotides of any length, either ribonucleotides or deoxyribonucleotides. Thus, term "polynucleotide" encompasses single-stranded DNA, double-stranded DNA, multi-stranded DNA, single-stranded RNA, double-stranded RNA, multi-stranded RNA, genomic DNA, cDNA, DNA-RNA hybrids, and a polymer comprising purine and pyrimidine bases or other natural, chemically or biochemically modified, non-natural, or derivative nucleotide bases.

The term "nucleotide sequence" also encompasses a nucleic acid and polynucleotide as defined above.

The term "gene" refers to a hereditary unit corresponding to a DNA sequence that occupies a specific location on a chromosome and that contains the genetic instruction for a characteristic(s) or trait(s) in an organism.

The term "gene editing" or "genome editing", used interchangeably herein, refers to a type of genetic engineering in which a DNA sequence is inserted, deleted, modified or replaced in the genome of a target cell. The basic mechanism involved in gene editing through RNP complex is the recognition of target genomic loci and the modification of the genome through homology-directed recombination (HDR) or non-homologous end joining (NHEJ).

The term "genome editing composition" refers to a composition that comprises the molecular machinery that is capable to insert, delete, modify or replace a DNA sequence in the genome of a target cell. In the context of the invention, the genome editing composition comprises a ribonucleoprotein (RNP) complex and eventually a donor sequence.

The term "ribonucleoprotein complex" or "RNP complex" refers to a molecule containing both protein and RNA. RNP complex is often used to describe a RNA-guided endonuclease bound to a guide RNA (gRNA), which together forms an active enzyme. For genome editing, RNP complex can be delivered as a pre-assembled RNP complex, i.e. a RNA-guided endonuclease and a gRNA, or as a nucleic acid comprising a nucleotide sequence encoding the RNA-guided endonuclease and a nucleic acid comprising a nucleotide sequence encoding the gRNA.

The term "RNA-guided endonuclease" refers to an oligonucleotide-dependent enzyme which possesses catalytic activity for nucleic acid cleavage (e.g., ribonuclease activity) (ribonucleic acid cleavage), deoxyribonuclease activity (deoxyribonucleic acid cleavage), etc.). Exemplary RNA-guided endonuclease which may be used in accordance with the present teachings includes MAD7, CAS9, CAS12, Cpf 1, C2c1, C2c2.

As used herein, the term "cleavage" refers to the breakage of the covalent backbone of a nucleic acid molecule. Both single-stranded cleavage and double-stranded cleavage are possible, and double-stranded cleavage can occur as a result of two distinct single-stranded cleavage events.

The RNA-guided endonuclease is attached to a guide RNA (gRNA) to cleave the nucleic acid molecule, such as the genomic DNA, in a sequence specific manner.

The term "guide RNA" or "gRNA" refers to a ribonucleic acid comprising two segments: (i) a first segment (referred to herein as a "targeting segment" or "crRNA"), and (ii) a second segment (referred to herein as a "endonuclease-binding segment" or "tracrRNA").

By "segment" it is meant a segment/section/region of a molecule, e.g., a contiguous stretch of nucleotides in a nucleic acid molecule. A segment can also mean a region/section of a complex such that a segment may comprise regions of more than one molecule.

The "targeting segment" is also referred to herein as a "variable region" of a gRNA. The "endonuclease-binding segment" is also referred to herein as a "constant region" of a gRNA. A "targeting segment" of a gRNA comprises a guide sequence. The "guide sequence" (also referred to as the "targeting sequence") can be modified so that the gRNA can target any desired sequence of any desired target nucleic acid, with the exception that the protospacer adjacent motif (PAM) sequence can be taken into account.

The term "hybridisable" or "complementary" refers to a nucleic acid (e.g. RNA, DNA) comprises a sequence of nucleotides that enables it to non- covalently bind, i.e. form Watson-Crick base pairs and/or G/U base pairs, "anneal", or "hybridize", to another nucleic acid in a sequence-specific, antiparallel, manner (i.e., a nucleic acid specifically binds to a complementary nucleic acid) under the appropriate in vitro and/or in vivo conditions of temperature and solution ionic strength. Standard Watson-Crick base-pairing includes: adenine (A) pairing with thymidine (T), adenine (A) pairing with uracil (U), and guanine (G) pairing with cytosine (C) [DNA, RNA]. Guanine (G) can also base pair with uracil (U). Hybridization requires that the two nucleic acids contain complementary sequences, although mismatches between bases are possible. The conditions appropriate for hybridization between two nucleic acids depend on the length of the nucleic acids and the degree of complementarity, variables well known in the art. The greater the degree of complementarity between two nucleotide sequences, the greater the value of the melting temperature (Tm) for hybrids of nucleic acids having those sequences. For hybridizations between nucleic acids with short stretches of complementarity (e.g. complementarity over 35 or fewer, 30 or fewer, 25 or fewer, 22 or fewer, 20 or fewer, or 18 or fewer nucleotides) the position of mismatches can become important. Typically, the length for a hybridizable nucleic acid is 8 nucleotides or more (e.g., 10 nucleotides or more, 12 nucleotides or more, 15 nucleotides or more, 20 nucleotides or more, 22 nucleotides or more, 25 nucleotides or more, or 30 nucleotides or more).

The term "donor nucleic acid" refers to a nucleic acid sequence to be inserted at the site cleaved by a RNP complex (e.g., after dsDNA cleavage, after nicking a target DNA, after dual nicking a target DNA, and the like). The donor nucleic acid can contain sufficient homology to a genomic sequence at the target site, e.g. 70%, 80%, 85%, 90%, 95%, 96%, 97%, 98%, 99% or 100% homology with the nucleotide sequences flanking the target site, e.g. within about 50 bases or less of the target site, e.g. within about 30 bases, within about 15 bases, within about 10 bases, within about 5 bases, or immediately flanking the target site, to support homology-directed repair between it and the genomic sequence to which it bears homology. Approximately 25, 50, 100, or 200 nucleotides, or more than 200 nucleotides, of sequence homology between a donor nucleic acid and a genomic sequence (or any integral value between 10 and 200 nucleotides, or more) can support homology-directed repair. Donor nucleic acid can be of any length, e.g. 10 nucleotides or more, 50 nucleotides or more, 100 nucleotides or more, 250 nucleotides or more, 500 nucleotides or more, 1000 nucleotides or more, 5000 nucleotides or more, etc. In some embodiments, the donor nucleic acid has a length of from 50 nucleotides to 100 nucleotides. In some cases, the donor nucleic acid has a length of from 50 nucleotides (nt) to 60 nt, from 60 nt to 70 nt, from 70 nt to 80 nt, form 80 nt to 90 nt, or from 90 nt to 100 nt. The donor nucleic acid is typically not identical to the genomic sequence that it replaces. Rather, the donor sequence may contain at least one or more single base changes, insertions, deletions, inversions or rearrangements with respect to the genomic sequence, so long as sufficient homology is present to support homology-directed repair.

The term "sonication" refers to the act of applying sound energy to agitate particles in a sample. Ultrasonic frequencies (≥ 20 kHz) are usually used, leading to the use of the term "ultrasonication". The sonication is usually applied using an ultrasonic bath or an ultrasonic probe, colloquially known as a sonicator.

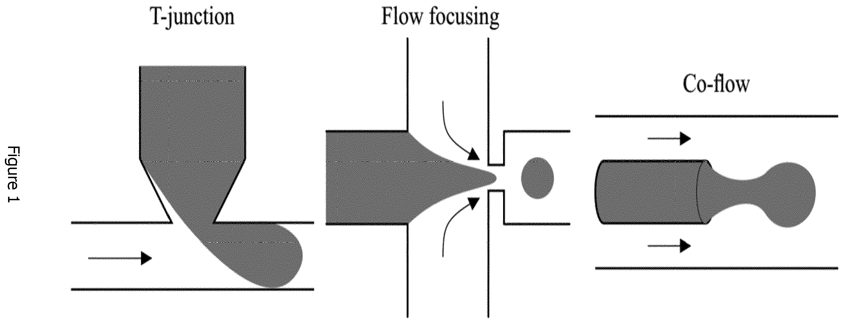

The term "microfluidic microdroplets generator" refers to a microfluidic device that generates microdroplets. The key principle of a "microfluidic microdroplets generator" is to use at least two streams of immiscible fluids (or immiscible phases) and to create a shear force on one of the phases in order to break the stream into discrete microdroplets. In some embodiments, the microfluidic microdroplets generator may be a "microfluidic flow-focusing microdroplets generator", a "microfluidic T junction microdroplets generator" or a "microfluidic co-flow microdroplets generator" (Figure 1).

In a "microfluidic flow-focusing microdroplets generator", the middle phase is squeezed between two streams of the continuous phase. In a "microfluidic T junction microdroplets generator", two immiscible phases are injected (generally with a pressure controller) in two orthogonal channels; the droplet formation occurs at the intersection of the two channels. In a "microfluidic co-flow microdroplets generator", two immiscible phases are injected (generally with a pressure controller) in two parallel channels, one channel within the other channel; the droplet formation occurs at the intersection of the two channels.

The term "microdroplets" refers to droplets generated with a microfluidic microdroplets generator.

The term "lipid microdroplets" refers to microdroplets comprising an aqueous phase encapsulated in a lipid phase.

The term "plant" refers to whole plants, a grafted plant, ancestors and progeny of the plants and plant parts, including seeds, shoots, stems, roots (including tubers), rootstock, scion, and plant cells, tissues and organs. The plant may be in any form including suspension cultures, embryos, meristematic regions, callus tissue, leaves, gametophytes, sporophytes, pollen, and microspores.

The term "cannabis" refers to the genus which includes all different species including *Cannabis sativa, Cannabis indica* and *Cannabis ruderalis* as well as wild Cannabis.

The term "cucurbita" refers to the genus which includes all different species including *Cucumis sativus, Citrullus lanatus, Cucumis melo, Cucurbita maxima.*

The term "luminescent dye" refers to a chemical compound that exhibits luminescence. There are three types of luminescent dye: fluorescent dye, phosphorescent dye and radioluminescent dye. For example, the term "fluorescent dye" or "fluorophore" refers to a fluorescent chemical compound that can re-emit light upon light excitation.

The term "pollinating a plant ovule" refers to the transfer of pollen grains to the stigma of a plant, later enabling fertilization and the production of seeds.

The term "gene edited homozygous plant", by opposition to "gene edited heterozygous plant", refers to a plant having the two alleles of a target locus edited.

### Method of transforming and/or gene editing pollen grains

As disclosed herein, Clustered Regularly Interspersed Short Palindromic Repeats/CRISPR-associated system (e.g. CRISPR/Cas system) is used for plant genome editing. The CRISPR associated system provides a relatively simple, effective tool for generating modifications in genomic DNA at target sites. CRISPR/Cas system can be used to create targeted double-strand or single-strand breaks, and can be used for, without limitation, targeted mutagenesis, gene targeting, gene replacement, targeted deletions, targeted inversions, targeted translocations, targeted insertions, and multiplexed genome modification. This technology can be used to accelerate the rate of functional genetic studies in plants, and to engineer plants with improved characteristics, including enhanced nutritional quality, increased resistance to disease and stress, and heightened production of commercially valuable compounds.

A first object of the invention relates to a method of transforming pollen grains, the method comprising the steps of:
(a) providing pollen grains having at least one pore,
(b) providing a genome editing composition, the composition comprising a ribonucleoprotein (RNP) complex, said RNP complex comprising (i) a RNA-guided endonuclease or a nucleic acid comprising a nucleotide sequence encoding the RNA-guided endonuclease, and (ii) a guide RNA (gRNA) or a nucleic acid comprising a nucleotide sequence encoding the gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby obtaining transformed pollen grains.

In some embodiments of the first object of the invention, the method of transforming pollen grains comprises the steps of:
(a) providing pollen grains having at least one pore,
(b) providing a genome editing composition, the composition comprising a ribonucleoprotein (RNP) complex, said RNP complex comprising (i) a RNA-guided endonuclease, and (ii) a guide RNA (gRNA),
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby obtaining transformed pollen grains.

In some other embodiments of the first object of the invention, the method of transforming pollen grains comprises the steps of:
(a) providing pollen grains having at least one pore,
(b) providing a genome editing composition, the composition comprising a ribonucleoprotein (RNP) complex, said RNP complex comprising (i) a nucleic acid comprising a nucleotide sequence encoding a RNA-guided endonuclease, and (ii) a nucleic acid comprising a nucleotide sequence encoding a gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby obtaining transformed pollen grains.

In some other embodiments of the first object of the invention, the method of transforming pollen grains comprises the steps of:
(a) providing pollen grains having at least one pore,
(b) providing a genome editing composition, the composition comprising a ribonucleoprotein (RNP) complex, said RNP complex comprising (i) a RNA-guided endonuclease, and (ii) a nucleic acid comprising a nucleotide sequence encoding a gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby obtaining transformed pollen grains.

In some other embodiments of the first object of the invention, the method of transforming pollen grains comprises the steps of:
(a) providing pollen grains having at least one pore,
(b) providing a genome editing composition, the composition comprising a ribonucleoprotein (RNP) complex, said RNP complex comprising (i) a nucleic acid comprising a nucleotide sequence encoding a RNA-guided endonuclease, and (ii) a guide RNA (gRNA),
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby obtaining transformed pollen grains.

A second object of the invention relates to a method of gene editing pollen grains, the method comprising the steps of:
(a) providing pollen grains having at least one pore,
(b) providing a genome editing composition, the composition comprising a ribonucleoprotein (RNP) complex, said RNP complex comprising (i) a RNA-guided endonuclease or a nucleic acid comprising a nucleotide sequence encoding the RNA-guided endonuclease, and (ii) a guide RNA (gRNA) or a nucleic acid comprising a nucleotide sequence encoding the gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby generating gene edited pollen grains.

In some embodiments of the second object of the invention, the method of gene editing pollen grains comprises the steps of:
(a) providing pollen grains having at least one pore,
(b) providing a genome editing composition, the composition comprising a ribonucleoprotein (RNP) complex, said RNP complex comprising (i) a RNA-guided endonuclease, and (ii) a guide RNA (gRNA),
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby generating gene edited pollen grains.

In some embodiments of the second object of the invention, the method of gene editing pollen grains comprises the steps of:
(a) providing pollen grains having at least one pore,
(b) providing a genome editing composition, the composition comprising a ribonucleoprotein (RNP) complex, said RNP complex comprising (i) a nucleic acid comprising a nucleotide sequence encoding a RNA-guided endonuclease, and (ii) a nucleic acid comprising a nucleotide sequence encoding a gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby generating gene edited pollen grains.

In some embodiments of the second object of the invention, the method of gene editing pollen grains comprises the steps of:
(a) providing pollen grains having at least one pore,
(b) providing a genome editing composition, the composition comprising a ribonucleoprotein (RNP) complex, said RNP complex comprising (i) a RNA-guided endonuclease, and (ii) a nucleic acid comprising a nucleotide sequence encoding a gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby generating gene edited pollen grains.

In some embodiments of the second object of the invention, the method of gene editing pollen grains comprises the steps of:
(a) providing pollen grains having at least one pore,
(b) providing a genome editing composition, the composition comprising a ribonucleoprotein (RNP) complex, said RNP complex comprising (i) a nucleic acid comprising a nucleotide sequence encoding a RNA-guided endonuclease, and (ii) a guide RNA (gRNA),
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby generating gene edited pollen grains.

### Step a)

Step a) is providing pollen grains having at least one pore.

In some embodiments, the pollen grains are collected from a plant producing pollen grains having at least one pore, for example a plant of the poaceae family, such as a plant of the genus oryza or avena. The plant may be, for example, selected from a Cannabis, a Cucurbita or a Zea. In some embodiments, the pollen grain is from *cannabis sativa.*

The collected pollen grains can be immediately used for transformation and/or gene editing, or it can be stored under conditions which will substantially preserve its viability and quality. To be effective for transformation and/or gene editing techniques, not only must the pollen grains viability be maintained, but the pollen grains tube must maintain its ability to elongate through the style of the flower to reach the ovules. For example, pollen grains can be stored within the range of -70°C to -20°C, -60°C to -30°C, and -50°C to -40°C. Pollen grains can also be stored in a dry environment at room temperature after drying said pollen grains. ,

In some embodiments, the pollen grains are in a solution, e.g. a pollen grains suspension. Therefore, step a) may be: (i) providing a suspension of pollen grains having at least one pore or (ii) mixing pollen grains having at least one pore in a solution thereby obtaining a suspension of pollen grains. The solution in which the pollen grains are mixed can be selected by the skilled person for its properties (i) to prevent hemp pollen grains aggregation, (ii) to allow the preparation of the microdroplets in step d), in particular the preparation of the microdroplets using a microfluidic microdroplets generator (see below), and/or (iii) to provide a good transformation rate.

### Step b)

Step b) is providing a genome editing composition, the composition comprising a ribonucleoprotein (RNP) complex, said RNP complex comprising (i) a RNA-guided endonuclease or a nucleic acid comprising a nucleotide sequence encoding the RNA-guided endonuclease, and (ii) a guide RNA (gRNA) or a nucleic acid comprising a nucleotide sequence encoding the gRNA.

The genome editing composition is in a form suitable for the preparation of the microdroplets in step d), in particular in a form suitable for the preparation of the microdroplets using a microfluidic microdroplets generator (see below). Therefore, the genome editing composition is preferably in a liquid form.

The genome editing composition can be prepared by mixing a RNP complex comprising (i) a RNA-guided endonuclease or a nucleic acid comprising a nucleotide sequence encoding the RNA-guided endonuclease, and (ii) a guide RNA (gRNA) or a nucleic acid comprising a nucleotide sequence encoding the gRNA.

In a preferred embodiment, the nucleic acid comprising a nucleotide sequence encoding a RNA-guided endonuclease and the nucleic acid comprising a nucleotide sequence encoding the gRNA gene do not insert into the genome of the pollen grain. The nucleotide sequences are therefore non-integrative nucleotide sequences.

In some embodiments, the RNP complex comprises (i) a RNA-guided endonuclease, and (ii) a guide RNA (gRNA). In this embodiment, the genome editing composition can be prepared by mixing the RNA-guided endonuclease and the guide RNA (gRNA).

In some other embodiments, the RNP complex comprises (i) a nucleic acid comprising a nucleotide sequence encoding a RNA-guided endonuclease, and (ii) a nucleic acid comprising a nucleotide sequence encoding the gRNA. The RNP complex may be one non-integrative vector, such as one plasmid, comprising both (i) the nucleic acid comprising a nucleotide sequence encoding a RNA-guided endonuclease, and (ii) the nucleic acid comprising a nucleotide sequence encoding the gRNA. The RNP complex may be two non-integrative vectors, such as two plasmids: one non-integrative vector comprising the nucleic acid comprising a nucleotide sequence encoding a RNA-guided endonuclease, and another non-integrative vector comprising the nucleic acid comprising a nucleotide sequence encoding the gRNA. Therefore, the genome editing composition can be prepared by mixing the two non-integrative vectors.

In some other embodiments, the RNP complex comprises (i) a RNA-guided endonuclease, and (ii) a nucleic acid comprising a nucleotide sequence encoding the gRNA. The RNP complex may be (i) the RNA-guided endonuclease, and (ii) one non-integrative vector, such as one plasmid, comprising the nucleic acid comprising a nucleotide sequence encoding the gRNA. In this embodiment, the genome editing composition can be prepared by mixing the RNA-guided endonuclease and the nucleic acid comprising a nucleotide sequence encoding the gRNA.

In some other embodiments, the RNP complex comprises (i) a nucleic acid comprising a nucleotide sequence encoding the RNA-guided endonuclease, and (ii) a guide RNA (gRNA). The RNP complex may be (i) one non-integrative vector, such as one plasmid, comprising the nucleic acid comprising a nucleotide sequence encoding the RNA-guided endonuclease, and (ii) the guide RNA (gRNA). In this embodiment, the genome editing composition can be prepared by mixing the nucleic acid comprising a nucleotide sequence encoding the RNA-guided endonuclease and the guide RNA (gRNA).

It has to be understood that when the RNP complex comprises a nucleic acid comprising a nucleotide sequence encoding a RNA-guided endonuclease, said RNA-guided endonuclease is produced within the pollen grain, i.e. using the cellular machinery of the pollen grain for protein production. Also, it has to be understood that when the RNP complex comprises a nucleic acid comprising a nucleotide sequence encoding the gRNA, said gRNA is produced within the pollen grain, using the cellular machinery of the pollen grain for RNA production.

In a preferred embodiment, the RNP complex comprises (i) a RNA-guided endonuclease, and (ii) a guide RNA (gRNA). This embodiment is particularly interesting because there is no need to transcript and/or translate a nucleic acid before editing the genome of the pollen grains. Therefore, the pollen grains can be gene edited as soon as the RNP complex is in contact with the genome of the pollen grains.

In some embodiments, the gRNA is a single-molecule (or "single guide") guide RNA (an "sgRNA"), i.e. the gRNA is a crRNA-tracrRNA chimera. In some embodiments, the gRNA is a dual-molecule (or "dual-guide") gRNA ("dgRNA"), also known as "crRNA-tracrRNA duplex", i.e. the crRNA and tracrRNA are two independent RNA molecules.

In some embodiments, the gRNA (e.g., sgRNA) has a total length of from 35 nucleotides (nt) to 150 nt. In some embodiments, the gRNA (e.g., the sgRNA) has a total length of from 35 nt to 40 nt, from 40 nt to 45 nt, from 45 nt to 50 nt, from 50 nt to 60 nt, from 60 nt to 70 nt, from 70 nt to 80 nt, from 80 nt to 90 nt, from 90 nt to 100 nt, from 100 nt to 125 nt, or from 125 nt to 150 nt.

The targeting segment of the gRNA (e.g., the sgRNA) can have a length of 7 or more nucleotides (nt) (e.g., 8 or more, 9 or more, 10 or more, 12 or more, 15 or more, 20 or more, 25 or more, 30 or more, or 40 or more nucleotides). In some embodiments, the targeting segment can have a length of from 7 to 100 nucleotides (nt) (e.g., from 7 to 80 nt, from 7 to 60 nt, from 7 to 40 nt, from 7 to 30 nt, from 7 to 25 nt, from 7 to 22 nt, from 7 to 20 nt, from 7 to 18 nt, from 8 to 80 nt, from 8 to 60 nt, from 8 to 40 nt, from 8 to 30 nt, from 8 to 25 nt, from 8 to 22 nt, from 8 to 20 nt, from 8 to 18 nt, from 10 to 100 nt, from 10 to 80 nt, from 10 to 60 nt, from 10 to 40 nt, from 10 to 30 nt, from 10 to 25 nt, from 10 to 22 nt, from 10 to 20 nt, from 10 to 18 nt, from 12 to 100 nt, from 12 to 80 nt, from 12 to 60 nt, from 12 to 40 nt, from 12 to 30 nt, from 12 to 25 nt, from 12 to 22 nt, from 12 to 20 nt, from 12 to 18 nt, from 14 to 100 nt, from 14 to 80 nt, from 14 to 60 nt, from 14 to 40 nt, from 14 to 30 nt, from 14 to 25 nt, from 14 to 22 nt, from 14 to 20 nt, from 14 to 18 nt, from 16 to 100 nt, from 16 to 80 nt, from 16 to 60 nt, from 16 to 40 nt, from 16 to 30 nt, from 16 to 25 nt, from 16 to 22 nt, from 16 to 20 nt, from 16 to 18 nt, from 18 to 100 nt, from 18 to 80 nt, from 18 to 60 nt, from 18 to 40 nt, from 18 to 30 nt, from 18 to 25 nt, from 18 to 22 nt, or from 18 to 20 nt).

It has to be understood that the invention should not be limited to targeting a specific gene, since the method of the invention can be easily adapted to any target sequence without difficulty by the skilled person. A skilled person will have no difficulty to design an appropriate gRNA for targeting a specific gene of the pollen grain, and altering its expression, for example, but not limited to, any gene listed in Table 1.

In some embodiments, the gRNA comprises a nucleotide sequence that hybridizes with a contiguous stretch of from about 7 nucleotides (nt) to about 50 nt (e.g, 7 nt, 8, nt, 9 nt, 10 nt, from 10 nt to 15 nt, from 15 nt to 20 nt, from 20 nt to 25 nt, from 25 nt to 30 nt, from 30 nt to 35 nt, from 35 nt to 40 nt, form 40 nt to 45 nt, or from 45 nt to 50 nt) of a nucleotide sequence that is involved in expressing a gene listed in Table 1, such as the nucleotide sequence of a gene itself, its promoter sequence, etc.

**Table 1: Examples of target genes**

| **gene_set** | **gene_id** | **products** |
|---|---|---|
| Disease Resistance | 115705883 | [MLO-like protein 12] |
| Disease Resistance | 115706217 | [MLO-like protein 11] |
| Disease Resistance | 115718602 | [MLO-like protein 3] |
| Disease Resistance | 115719652 | [MLO-like protein 6] |
| Disease Resistance | 115708720 | [MLO-like protein 3] |
| Disease Resistance | 115714309 | [MLO-like protein 8] |
| Flowering Time | 115712529 | [zinc finger protein JAGGED isoform X1,zinc finger protein JAGGED isoform X2,zinc finger protein JAGGED, transcript variant X1,zinc finger protein JAGGED, transcript variant X2] |
| Disease Resistance | 115713054 | [MLO-like protein 13, transcript variant X1,MLO-like protein 13, transcript variant X2,MLO-like protein 13 isoform X1,MLO-like protein 13 isoform X2] |
| Disease Resistance | 115713722 | [MLO-like protein 9] |
| Disease Resistance | 115725262 | [MLO-like protein 4, transcript variant X1,MLO-like protein 4, transcript variant X2,MLO-like protein 4 isoform X1,MLO-like protein 4 isoform X2] |
| Disease Resistance | 115697278 | [MLO-like protein 6, transcript variant X1,MLO-like protein 6, transcript variant X2,MLO-like protein 6 isoform X1,MLO-like protein 6 isoform X2] |
| Disease Resistance | 115698914 | [MLO-like protein 12] |
| Flowering Time | 115703149 | [protein EARLY FLOWERING 3] |
| Disease Resistance | 115697297 | [MLO-like protein 1] |
| Disease Resistance | 115716111 | [MLO-like protein 1] |
| Disease Resistance | 115702656 | [MLO-like protein 4 isoform X1,MLO-like protein 4 isoform X2,MLO-like protein 4 isoform X3,MLO-like protein 4, transcript variant X4,MLO-like protein 4, transcript variant X1,MLO-like protein 4, transcript variant X3,MLO-like protein 4, transcript variant X2] |
| Auxin Response | 115703736 | [auxin-responsive protein SAUR32] |
| Auxin Response | 115703914 | [auxin-responsive protein SAUR76] |
| Auxin Response | 115707646 | [auxin-responsive protein SAUR36-like] |
| Auxin Response | 115704545 | [auxin-responsive protein SAUR71-like] |
| Auxin Response | 115704804 | [auxin-responsive protein SAUR32-like] |
| Auxin Response | 115704933 | [auxin-responsive protein SAUR78] |
| Auxin Response | 115708216 | [auxin-responsive protein SAUR36] |
| Auxin Response | 115706260 | [auxin-responsive protein SAUR32-like] |
| Auxin Response | 115721013 | [auxin-responsive protein SAUR21-like] |
| Auxin Response | 115720912 | [auxin-responsive protein SAUR50] |
| Auxin Response | 115721180 | [auxin-responsive protein SAUR21] |
| Auxin Response | 115719725 | [auxin-responsive protein SAUR21-like] |
| Auxin Response | 115708788 | [auxin-responsive protein SAUR71] |
| Auxin Response | 115711481 | [auxin-responsive protein SAUR71] |
| Auxin Response | 115710179 | [auxin-responsive protein SAUR78] |
| Auxin Response | 115710625 | [auxin-responsive protein SAUR50] |
| Auxin Response | 115714709 | [auxin-responsive protein SAUR50] |
| Auxin Response | 115714723 | [auxin-responsive protein SAUR36] |
| Auxin Response | 115716299 | [auxin-responsive protein SAUR36] |
| Auxin Response | 115718161 | [auxin-responsive protein SAUR32] |
| Auxin Response | 115724698 | [auxin-responsive protein SAUR21-like] |
| Auxin Response | 115695781 | [auxin-responsive protein SAUR24-like] |
| Auxin Response | 115725224 | [auxin-responsive protein SAUR50] |
| Auxin Response | 115725225 | [auxin-responsive protein SAUR21] |
| Auxin Response | 115724699 | [auxin-responsive protein SAUR23-like] |
| Auxin Response | 115725167 | [auxin-responsive protein SAUR21] |
| Auxin Response | 115725551 | [auxin-responsive protein SAUR24] |
| Auxin Response | 115725165 | [auxin-responsive protein SAUR21-like] |
| Auxin Response | 115725166 | [auxin-responsive protein SAUR21] |
| Auxin Response | 115724709 | [auxin-responsive protein SAUR21] |
| Auxin Response | 115695782 | [auxin-responsive protein SAUR24-like] |
| Auxin Response | 115725651 | [auxin-responsive protein SAUR24-like] |
| Auxin Response | 115695459 | [auxin-responsive protein SAUR15-like] |
| Auxin Response | 115698991 | [auxin-responsive protein SAUR71] |
| Auxin Response | 115701761 | [auxin-responsive protein SAUR50] |
| Auxin Response | 115702521 | [auxin-responsive protein SAUR68-like] |
| Auxin Response | 115702391 | [auxin-responsive protein SAUR24-like] |
| Auxin Response | 115707600 | [auxin-responsive protein SAUR15-like] |
| Auxin Response | 115707456 | [auxin-responsive protein SAUR21,auxin-responsive protein SAUR21, transcript variant X1,auxin-responsive protein SAUR21, transcript variant X2] |
| Auxin Response | 115702479 | [auxin-responsive protein SAUR68] |
| Specific Cannabinoid & Mix | 115715915 | [terpene synthase 10-like, transcript variant X1,terpene synthase 10-like, transcript variant X2,terpene synthase 10-like isoform X1,terpene synthase 10-like isoform X2] |
| Specific Cannabinoid & Mix | 115716805 | [terpene synthase 10] |
| Specific Cannabinoid & Mix | 115716806 | [terpene synthase 10-like, transcript variant X1,terpene synthase 10-like, transcript variant X2,terpene synthase 10-like, transcript variant X3,terpene synthase 10-like, transcript variant X4,terpene synthase 10-like isoform X1,terpene synthase 10-like isoform X2,terpene synthase 10-like isoform X3,terpene synthase 10-like isoform X4] |
| Specific Cannabinoid & Mix | 115715811 | [terpene synthase 10] |
| Specific Cannabinoid & Mix | 115699029 | [probable terpene synthase 11] |
| Specific Cannabinoid & Mix | 115701179 | [probable terpene synthase 9] |
| Specific Cannabinoid & Mix | 115723097 | [probable monoterpene synthase MTS1, chloroplastic] |
| Specific Cannabinoid & Mix | 115723096 | [probable monoterpene synthase MTS1, chloroplastic] |
| Specific Cannabinoid & Mix | 115722765 | [probable monoterpene synthase MTS1, chloroplastic] |
| Specific Cannabinoid & Mix | 115723095 | [probable monoterpene synthase MTS1, chloroplastic] |
| Transcription Factor | 115707341 | [WUSCHEL-related homeobox 5] |
| Transcription Factor | 115720942 | [WUSCHEL-related homeobox 3] |
| Transcription Factor | 115720364 | [WUSCHEL-related homeobox 2-like] |
| Transcription Factor | 115711960 | [protein WUSCHEL] |
| Transcription Factor | 115697124 | [WUSCHEL-related homeobox 9] |
| Transcription Factor | 115724016 | [WUSCHEL-related homeobox 11-like] |
| Transcription Factor | 115711571 | [WUSCHEL-related homeobox 8] |
| Transcription Factor | 115702606 | [WUSCHEL-related homeobox 8] |
| Transcription Factor | 115712206 | [WUSCHEL-related homeobox 4] |
| Light Regulation | 115707127 | [protein PHYTOCHROME-DEPENDENT LATE-FLOWERING] |
| Light Regulation | 115719277 | [phytochrome A] |
| Light Regulation | 115721362 | [phytochromobilin:ferredoxin oxidoreductase, chloroplastic] |
| Light Regulation | 115708831 | [protein PHYTOCHROME KINASE SUBSTRATE 3] |
| Light Regulation | 115716538 | [phytochrome-interacting ankyrin-repeat protein 2] |
| Light Regulation | 115694905 | [phytochrome-interacting ankyrin-repeat protein 2-like] |
| Light Regulation | 115697682 | [phytochrome-interacting ankyrin-repeat protein 2-like,LOW QUALITY PROTEIN: phytochrome-interacting ankyrin-repeat protein 2-like] |
| Light Regulation | 115697533 | [phytochrome E] |
| Light Regulation | 115698543 | [protein PHYTOCHROME KINASE SUBSTRATE 1] |
| Light Regulation | 115723926 | [protein PHYTOCHROME KINASE SUBSTRATE 4, transcript variant X1,protein PHYTOCHROME KINASE SUBSTRATE 4, transcript variant X2,protein PHYTOCHROME KINASE SUBSTRATE 4 isoform X1,protein PHYTOCHROME KINASE SUBSTRATE 4 isoform X2] |
| Light Regulation | 115721719 | [phytochrome B, transcript variant X1,phytochrome B, transcript variant X2,phytochrome B isoform X1,phytochrome B isoform X2] |
| Light Regulation | 115695743 | [LOW QUALITY PROTEIN: phytochrome A-associated F-box protein-like,phytochrome A-associated F-box protein-like] |
| Light Regulation | 115695661 | [phytochrome A-associated F-box protein] |
| Light Regulation | 115709957 | [cryptochrome DASH, chloroplastic/mitochondrial,cryptochrome DASH, chloroplastic/mitochondrial, transcript variant X2,cryptochrome DASH, chloroplastic/mitochondrial, transcript variant X1] |
| Light Regulation | 115722889 | [cryptochrome-1, transcript variant X1,cryptochrome-1, transcript variant X2,cryptochrome-1, transcript variant X3,cryptochrome-1, transcript variant X4,cryptochrome-1 isoform X2,cryptochrome-1 isoform X3,cryptochrome-1 isoform X1] |
| Light Regulation | 115699248 | [cryptochrome-1] |
| Specific Cannabinoid & Mix | 115717841 | [cannabidiolic acid synthase-like] |
| Specific Cannabinoid & Mix | 115697126 | [cannabidiolic acid synthase-like 1] |
| Specific Cannabinoid & Mix | 115697019 | [cannabidiolic acid synthase-like 1] |
| Specific Cannabinoid & Mix | 115697762 | [cannabidiolic acid synthase-like,cannabidiolic acid synthase] |
| Specific Cannabinoid & Mix | 115696987 | [cannabidiolic acid synthase-like] |
| Specific Cannabinoid & Mix | 115696884 | [cannabidiolic acid synthase-like 1] |
| Specific Cannabinoid & Mix | 115718836 | [tetrahydrocannabinolic acid synthase-like] |
| Specific Cannabinoid & Mix | 115720716 | [tetrahydrocannabinolic acid synthase-like] |
| Specific Cannabinoid & Mix | 115696986 | [tetrahydrocannabinolic acid synthase-like] |
| Specific Cannabinoid & Mix | 115697886 | [inactive tetrahydrocannabinolic acid synthase-like] |
| Specific Cannabinoid & Mix | 115698060 | [inactive tetrahydrocannabinolic acid synthase-like] |
| Specific Cannabinoid & Mix | 115696909 | [inactive tetrahydrocannabinolic acid synthase-like] |
| Specific Cannabinoid & Mix | 115697880 | [inactive tetrahydrocannabinolic acid synthase] |
| Auxin Response | 115707648 | [ARF guanine-nucleotide exchange factor GNOM,ARF guanine-nucleotide exchange factor GNOM, transcript variant X1,ARF guanine-nucleotide exchange factor GNOM, transcript variant X2] |
| Auxin Response | 115705675 | [auxin response factor 2B] |
| Auxin Response | 115705687 | [LOW QUALITY PROTEIN: auxin response factor 2B-like,auxin response factor 2B-like] |
| Auxin Response | 115706063 | [auxin response factor 16] |
| Auxin Response | 115706103 | [auxin response factor 9] |
| Auxin Response | 115704658 | [ARF guanine-nucleotide exchange factor GNOM isoform X1,ARF guanine-nucleotide exchange factor GNOM isoform X2,ARF guanine-nucleotide exchange factor GNOM, transcript variant X2,ARF guanine-nucleotide exchange factor GNOM, transcript variant X1] |
| Auxin Response | 115705617 | [auxin response factor 19 isoform X1,auxin response factor 7 isoform X2,auxin response factor 19, transcript variant X5,auxin response factor 19, transcript variant X2,auxin response factor 19, transcript variant X3,auxin response factor 19, transcript variant X1,auxin response factor 19, transcript variant X4] |
| Auxin Response | 115705861 | [LOW QUALITY PROTEIN: auxin response factor 18-like,auxin response factor 18-like] |
| Auxin Response | 115719139 | [auxin response factor 4, transcript variant X1,auxin response factor 4, transcript variant X2,auxin response factor 4 isoform X1,auxin response factor 4 isoform X2] |
| Auxin Response | 115718756 | [auxin response factor 16] |
| Auxin Response | 115719255 | [auxin response factor 10, transcript variant X1,auxin response factor 10, transcript variant X2,auxin response factor 10] |
| Auxin Response | 115709608 | [auxin response factor 19] |
| Auxin Response | 115709736 | [auxin response factor 18] |
| Auxin Response | 115714321 | [auxin response factor 1 isoform X1,auxin response factor 1 isoform X2,auxin response factor 1, transcript variant X1,auxin response factor 1, transcript variant X2,auxin response factor 1, transcript variant X5,auxin response factor 1, transcript variant X6,auxin response factor 1, transcript variant X3,auxin response factor 1, transcript variant X4] |
| Auxin Response | 115716126 | [auxin response factor 9] |
| Auxin Response | 115716528 | [ARF guanine-nucleotide exchange factor GNL2] |
| Auxin Response | 115694957 | [auxin response factor 18-like] |
| Auxin Response | 115694956 | [auxin response factor 18-like] |
| Auxin Response | 115694964 | [auxin response factor 18-like] |
| Auxin Response | 115697832 | [auxin response factor 6, transcript variant X1,auxin response factor 6, transcript variant X2,auxin response factor 6, transcript variant X3,auxin response factor 6] |
| Auxin Response | 115697396 | [auxin response factor 3] |
| Auxin Response | 115699476 | [auxin response factor 6] |
| Auxin Response | 115701709 | [auxin response factor 5 isoform X1,auxin response factor 5 isoform X2,auxin response factor 5, transcript variant X2,auxin response factor 5, transcript variant X1] |
| Auxin Response | 115703019 | [auxin response factor 2A,auxin response factor 2A, transcript variant X2,auxin response factor 2A, transcript variant X3,auxin response factor 2A, transcript variant X1] |
| Pigment biosynthesis | 115722384 | phytoene desaturase |

The gRNA can be complementary to target sites or target loci in the plant genome. Therefore, the nucleic acid sequence of the gRNA is designed depending on the target site to be gene edited. Therefore, according to the invention, the gRNA comprises a nucleic acid sequence that provides for correction and/or mutation of a target site. For example, a target site may be any target site that is involved in expressing a gene listed in Table 1, such as the nucleotide sequence of a gene itself, its promoter sequence, etc.. The skilled person will have no difficulty to design a gRNA comprising a suitable nucleic acid sequence depending on the target site to be gene edited.

In some embodiments, the ratio gRNA to RNA-guided endonuclease is from 100:1 to 1:1, such as from 50:1 to 2:1, such as from 10:1 to 2:1, preferably 5:1.

In some embodiment, the genome editing composition also comprises a nonviral nucleotide delivery reagent, for example polyethylenimine (PEI), nanoparticules, such as mesoporous silica nanoparticles (MSNs), liposome, modified magnetic metal particles, layered double hydroxides, carbon tubes. Therefore, the genome editing composition may be prepared by mixing a RNP complex with a nonviral nucleotide delivery reagent.

Polyethylenimine (PEI) is a stable hydrophilic cationic polymer that condenses proteins and/or nucleic acids into positively charged particles. In the context of the invention, PEI condenses the RNP complex into positively charged particles, which increases the transformation efficiency of pollen grains in step e).

In some embodiments, the RNP complex is labeled with a luminescent dye, such as a fluorescent dye. In one embodiment, the RNA-guided endonuclease is labeled with a luminescent dye, such as a fluorescent dye. In another embodiment, the gRNA is labeled with a luminescent dye, such as a fluorescent dye.

The fluorescent dye may be chosen from the group consisting of a cyanine, a rhodamine, a fluorescein, and derivatives thereof. For example, the cyanine may be chosen from the group consisting of Cy2, Cy3, Cy3B, Cy3.5, Cy5, Cy5.5 and Cy7, preferably Cy3 or Cy5. For example, the rhodamine may be chosen from the group consisting of rhodamine 6G, rhodamine 123, and rhodamine B. The fluorescent dye may also be a fluorescent peptide or a fluorescent protein, such as GFP (Green Fluorescent Protein), RFP (Red Fluorescent Protein) and derivatives thereof.

Methods for labelling RNA-guided endonuclease and/or gRNA are disclosed in the prior art (Masoud et al. 2019). In a specific embodiment, RNA-guided endonuclease is labelled with a N-HydroxySuccinimide (NHS) ester luminescent dye, for example chosen from the group consisting of NHS-Fluorescein, NHS-rhodamine and NHS-cyanine.

In some embodiments, the genome editing composition also comprises a donor nucleic acid. A genome editing composition comprising a donor nucleic acid enables RNA-guided genome editing in a site specific manner in the plant genome by insertion of the donor nucleic acid. Therefore, in some embodiments, the genome editing composition comprises (a) a RNP complex comprising (i) a RNA-guided endonuclease or a nucleic acid comprising a nucleotide sequence encoding the RNA-guided endonuclease, and (ii) a gRNA or a nucleic acid comprising a nucleotide sequence encoding the gRNA; and (b) a donor nucleic acid.

The donor nucleic acids can be complementary to target sites or target loci in the plant genome. Therefore, the nucleic acid sequence of the donor nucleic acid is designed depending on the target site to be gene edited. Therefore, according to the invention, the donor nucleic acid comprises a nucleic acid sequence that provides for correction and/or mutation of a target site. For example, the target site may be any target site that is involved in expressing a gene listed in Table 1, such as the nucleotide sequence of a gene itself, its promoter sequence, etc.. The skilled person will have no difficulty to design a donor nucleic acid comprising a suitable nucleic acid sequence depending on the target site to be gene edited.

### Step c)

Step c) is subjecting the pollen grains, such as the solution of pollen grains provided in step a), to sonication thereby obtaining sonicated pollen grains.

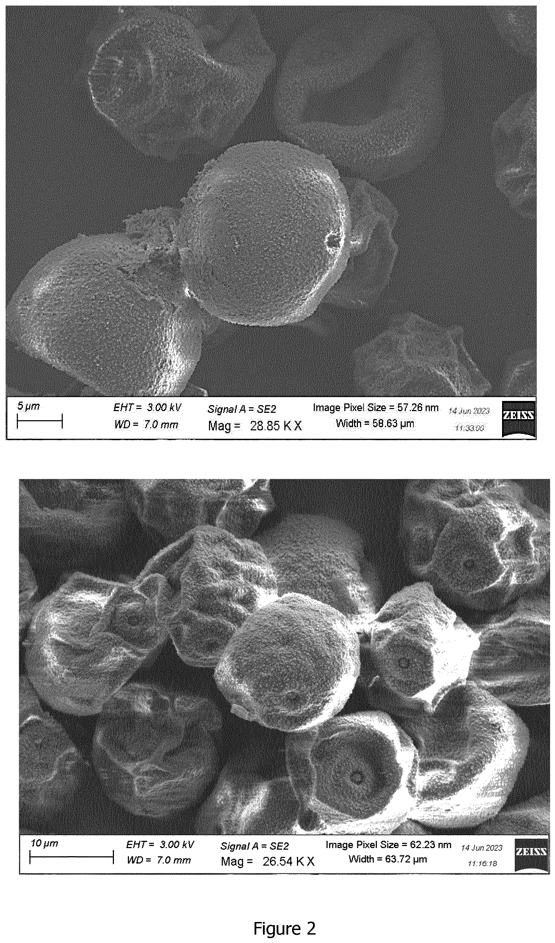

Pollen grains that are used in the present invention have germination pores on their surface sealed with opercula that prevent proteins and nucleic acid materials from diffusing through hemp pollen membranes in normal conditions. (Figure 2: microscopic photo of a hemp pollen grain and two germination pores). Sonication of pollen grains removes the opercula from the pollen grains. As a consequence, proteins and/or nucleic acids, such as the genome editing composition, can diffuse into the pollen grains through the germination pores. Thus, in the context of the invention, sonication increases the transformation rate of the pollen grains.

Another technical effect of sonication is to separate pollen grains that may remain stuck together, for example to disperse pollen grains in the solution of pollen grains.

In some embodiments, the sonication step is performed at a temperature below 22°C, preferably below 10°C, preferably at 4°C. For example, the recipient containing the pollen grains to be sonicated is kept on ice or surrounded by ethanol.

The sonication step may be performed with an ultrasonic homogenizer, for example by submerging the probe of the ultrasonic homogenizer into the solution of pollen grains.

The parameters of the sonication step are adapted to reduce alteration of viability of the pollen grains during sonication. In some embodiments, the sonication step is performed with (i) an amplitude from 100% to 10%, preferably from 50% to 10%, preferably 25%; (ii) a wattage from 200 Watts to 50 Watts, preferably from 150 Watts to 60 Watts, most preferably 120 Watts; and (iii) a frequency from 1 kHz to 100 kHz, preferably from 10 kHz to 50 kHz, most preferably 20 kHz. In a preferred embodiment, sonication is performed at a frequency of 20 kHz, at wattage of 120 Watts and amplitude of 25%. In some embodiments, the sonication is applied to the pollen grains for 1 second each time with an interval of 3 seconds for 90 seconds effective time. In some embodiments, sonication is performed with an amplitude of 30% , 120kHz a wattage of 120 Watts for 1 second each pulse with an interval of 3 seconds for 90 seconds effective time.

Pollen grains suspension was then treated with an ultrasonic homogenizer, on ice, by submerging the probe of the homogenizer into the pollen grains suspension, with 30% amplitude, 120W for 1 second each time with an interval of 3 second for 90 sec effective time

### Step d)

Step d) consists in incubating the sonicated pollen grains and the genome editing composition into microdroplets.

Incubation of the sonicated pollen grains and the genome editing composition into microdroplets allows manipulating very small and precise volumes of samples, but also allows realizing high throughput transformation and/or gene edition of pollen grains, as each microdroplets become a distinct micro-reactor. Moreover, microdroplets are a way to enhance mixing of pollen grains and a genome editing composition for increasing the rate of transformation and/or genome editing of pollen grains.

In some embodiments, the sonicated pollen grains and the genome editing composition are introduced into the microdroplets using a microfluidic microdroplets generator. Therefore, step d) may be: introduce the sonicated pollen grains and the genome editing composition into microdroplets using a microfluidic microdroplets generator and incubating the sonicated pollen grains and the genome editing composition into said microdroplets. In some embodiments, the microfluidic microdroplets generator may be a microfluidic flow-focusing microdroplets generator, a microfluidic T junction microdroplets generator or a microfluidic co-flow microdroplets generator. The microfluidic flow-focusing microdroplets generator is preferred because microdroplets formation is stable and microdroplets size and number are easily controlled.

Microdroplets increase the transformation efficiency because it increases the chance for the pollen grains to be in contact with the RNP complex in a small space and thus for the RNP complex to be incorporated into the pollen grains. As a consequence, it increases the genome editing efficiency. In some embodiments, the microdroplets are lipid microdroplets. Lipid microdroplets are commonly generated by microfluidic microdroplets generators.

For microfluidic flow-focusing microdroplets generators, the middle phase may comprise the sonicated pollen grains and the genome editing composition, and the two streams of the continuous phase may comprise a mixture of oil/surfactant.

For microfluidic T junction microdroplets generators, the continuous phase may comprise a mixture of oil/surfactant, and the orthogonal phase may comprise the sonicated pollen grains and the genome editing composition.

For microfluidic co-flow microdroplets generator, the inner phase may comprise the sonicated pollen grains and the genome editing composition, and the outer phase may comprise a mixture of oil/surfactant.

The skilled person will have no difficulty to select a mixture of oil/surfactant which is appropriate for generating microdroplets, depending on the microfluidic microdroplets generator. For example, the mixture oil/surfactant may be a mixture of a hydrofluoroethers (HFE), such as HFE 7500, and a fluorosurfactant, such as FC-50.

In some embodiments, the phase containing the pollen grains and the genome editing composition comprises from 25 uM to 5 uM of RNP complex (+/- donor nucleic acid), and from 1 mg to 10 mg of sonicated pollen grains. This allows obtaining microdroplets containing at least one pollen grain and at least one RNP complex.

### Step e)

Step e) is either (i) allowing penetration of the RNP complex into the sonicated pollen grains thereby obtaining transformed pollen grains, or (ii) allowing penetration of the RNP complex into the sonicated pollen grains thereby generating gene edited pollen grains.

The RNP complex and the sonicated pollen grains must be incubated into the microdroplets during a sufficient period of time to allow penetration of the RNP complex into the sonicated pollen grains.

In some embodiments, the sonicated pollen grains and the genome editing composition are incubated in the microdroplets for at least 30 minutes. In some embodiments, the sonicated pollen grains and the genome editing composition are incubated for about 30 minutes to about 1 hour.

### Optional step f)

In some embodiments, the method of the invention further comprises a step of screening the transformed pollen grains after step (e).The step of screening aims to (i) identify the microdroplets containing a pollen with RNP complex, such as a RNP complex labeled with a fluorescent dye, for example fluorescent screening using microscopy and (ii) select the microdroplets containing at least one pollen grain and at least one RNP complex, for example using FACS.

The transformed pollen grains and/or the gene edited pollen grains can then be used to pollinate a maternal plant, e.g. using any conventional techniques to spread the pollen on flowers, such as using a brush.

### Method of obtaining a gene edited homozygous plant

A third object of the invention relates to a method of obtaining a gene edited homozygous plant, the method comprising the step of pollinating a plant ovule with a pollen grain obtained by the method of transforming pollen grains according to the invention or a method of gene editing pollen grains according to the invention, thereby obtaining a gene edited homozygous plant.

Thanks to its stability, the RNP complex can remain in the transformed/gene edited pollen grains. In the context of the invention, the plant ovule genome is therefore gene edited during the fertilization with the transformed/gene edited pollen grain, thereby obtaining a gene edited homozygous plant embryo, thereby obtaining subsequently a gene edited homozygous seed, and thereby obtaining subsequently a gene edited homozygous complete plant.

In some embodiments, the time period between the transformation/gene edition of pollen grains and the pollination of the plant ovule should be sufficiently short to avoid degradation of the RNP complex. In some other embodiments, the transformed/gene edited pollen grains are stored in conditions that prevent either degradation of the RNP complex and alteration of viability of the pollen grains. For example, pollen grains can be stored within the range of - 70°C to -20°C, -60°C to -30°C, and -50°C to -40°C.

In some embodiments, prior to pollination, the microdroplets obtained in steps e) or f), containing transformed/gene edited pollen grains, are rinsed in a buffer, such as a germination buffer. In some embodiments, the rinsed transformed/gene edited pollen grains are mixed with a germination medium, such as a medium comprising sucrose, H₃BO₃, CaCl2, KH₂PO₄, PEG. An appropriate germination medium is disclosed in the examples and comprises 10% sucrose, 0.005% H₃BO₃, 10 mM CaCl₂, 0.05 mM KH₂PO₄, 6% PEG 4000. The resulting mixture can be directly applied on flowers for fertilization.

The homozygous seeds resulting from pollination of the plant ovule with transformed/gene edited pollen grains can be used to obtain complete, homozygous plants, without any remaining RNP complex, in one generation.

In some embodiments, the resulting seed and/or the resulting complete plant are examined for the presence of predicted genetic changes via sequencing or visual phenotypes.

Non-limiting advantages of the present method of obtaining a gene edited homozygous plant are listed below:
- No integration of a transgene.
- It is possible to obtain homozygous gene-edited plant in one generation.
- No regeneration and *in vitro* culture steps that potentially induce somatic mutation.
- Much faster method compared to conventional transformation methods.
- Can be applied on many plant species having porated pollen grains without major adjustments.

The invention will be further illustrated by the following figures and examples. However, these examples and figures should not be interpreted in any way as limiting the scope of the invention.

### DESCRIPTION OF THE FIGURES

**Figure 1****:** different types of microfluidic microdroplets generators suitable for introducing the sonicated pollen grains and the genome editing composition into microdroplets, namely T-junction microdroplets generator, flow focusing microdroplets generator and co-flow microdroplets generator.
**Figure 2****:** pollen grains of *Cannabis sativa* observed in electronic microscopy shows the presence of pores in the pollen grains membrane, said pores being obstructed by an operculum

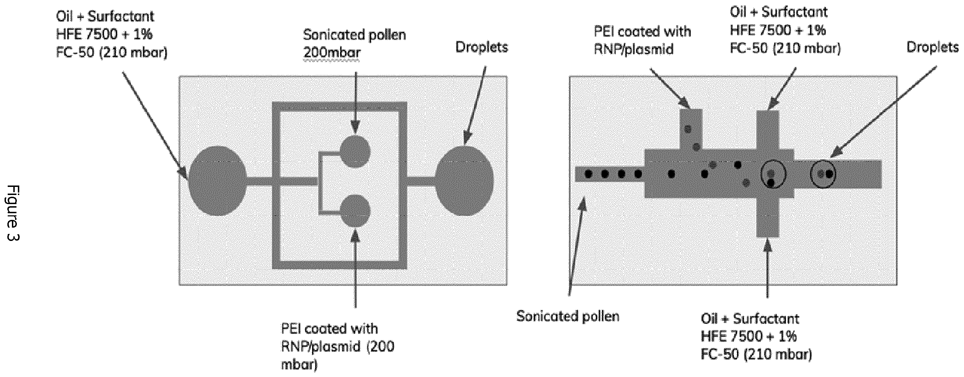

**Figure 3****:** complete structure of a microfluidic microdroplets generator suitable for introducing the sonicated pollen grains and the genome editing composition into microdroplets.

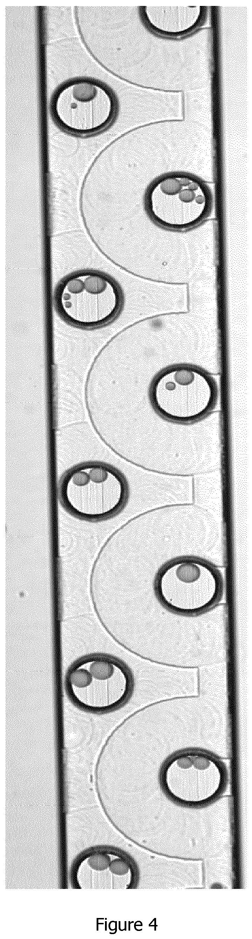

**Figure 4****:** microfluidic channel comprising microdroplets containing pollen grains.
**Figure 5****:** transformed pollen grains with the presence of labeled RNP particles in the tip shown by Cy5 fluorescence. The arrows show the fluorescence associated with the RNP.

### EXAMPLES

### Example 1: Materials and methods

### Plant material and pollen sampling

Pollen grains were collected from male plants of hemp (*Cannabis sativa*). Hemp seeds were sown in a soil mix of John Innes One, John Innes Two, Vermiculite, Perlite (1:1:1:1) into pots of 9x9x10cm and grown in a growth room under 24 hour blue/ red light. The pollen grains were collected from mature anthers by shaking male flowers and then dried for a week into paper-bag before using it.

### Pollen resuspension and aperture opening

10 mg of pollen grains were placed into a 5mL Eppendorf tube and resuspended in 4 mL of resuspension buffer and incubated for 30 minutes on ice. Pollen grains suspension was then treated with an ultrasonic homogenizer, on ice, by submerging the probe of the homogenizer into the pollen grains suspension, with 30% amplitude, 120W for 1 second each time with an interval of 3 second for 90 sec effective time. The suspension was then kept on ice until its use.

### MAD7 production and purification

MAD7 coding sequence was obtained from Inscripta and codon optimized for Nicotiana benthamiana and fused with a N-HydroxySuccinimide (NHS) Ester fluroflor (cyanine 5). The corresponding fragments were synthesized by Twist biosciences. MAD7 fragment was then subcloned into a pET28 plasmid under a T7 promoter. After transformation in E.coli DE3, colonies were screened by sequencing and a single positive clone was used for protein production. Starter culture was made by inoculating 5mL LB + Kanamycin 50 µg/mL with the positive clone and grew overnight at 37°C with 200 rpm shaking. Larger culture was made using the starter culture at 1:100 dilution and grew at 37°C with 200 rpm shaking. Once the OD600 reached 0.6-0.8, fresh IPTG (1mM) was added, the culture was grown for 5 more hours and the OD was checked. QC test was performed by spinning down an aliquot of 3mL at 15000g for 3 minutes. After removing supernatant, the pellet was resuspended in 300µL of PBS 1X and the crude lysate volume for gel loading was calculated based on OD (µL = 180/OD). After adding the appropriate volume of 4X LDS (NuPAGE LDS sample buffer 4X, ThermoFisher), DTT 50mM, MgCl₂ 100mM, the samples were heated at 72°C for 10 minutes, placed on ice for 5 minutes and then centrifuged for 15 minutes, 15000g. The supernatant was runned on SDS-PAGE gel (160V, 40-45 minutes) and the gel was revealed using Coomassie Blue. After QC test, the rest of the culture was spun down at 4°C, 7000 rpm for 5 minutes with no brake and the supernatant was removed. Cells were washed in resuspension buffer (50 mM Tris-HCl pH 8, 2 mM EDTA), spun down and the resulting pellet was snap frozen in liquid nitrogen and stored at 80°C . Proteins were then purified using Thermo Scientific^{™} HisPur^{™} Ni-NTA Spin Purification Kit according to the manufacturer instructions. TCA was added to the extract to a final concentration of 10-20% and the proteins were precipitated on ice for 30 min. After centrifugation, the pellet was washed with ethanol and then resuspended in nuclease buffer 50mM Na Phosphate pH7.5, 300 mM NaCl, 0.1mM EDTA, 20% glycerol, 1mM DTT, 3mM Ca(NO₃)₂ to a final concentration of 25µM.

### Guide RNA synthesis

The fragments containing sgRNA with their optimized scaffold (crRNA-sgRNA) under a minimal T7 promoter were ordered as forward and reverse fragments from Eurofins. Both fragments were annealed 5 min at 95°C and then incubated 20 minutes at room temperature. DNA fragments were then transcribed *in vitro* into RNA using MEGAscriptTM T7 from ThermoFisher, according to the manufacturer instructions. The obtained RNA fragments (gRNA) are dissolved in nuclease free water to a final concentration of 100uM.

### RNP construction

10µL of protein solution 25µM, 125 µL of guide RNA solution 100µM, PEI 1mg/mL to a ratio PEI/Protein of 4:1, qsp500 µL with Ca(NO₃)₂ 3mM were mixed together to obtain a ratio gRNA/MAD7 of 5:1.

### Microfluidic microdroplets generator

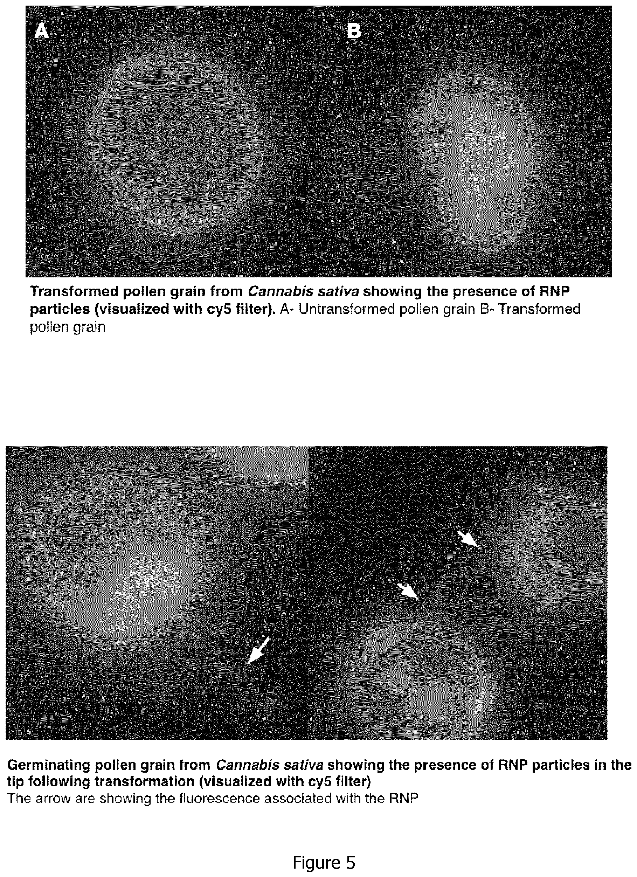

The Fluidic 719 chip was purchased from microfluidic chipshop. Oil (dSurf(fluigent) diluted to 2% in Novec7500(3M))was used as the continuous phase for the emulsification of the PEI/ RNP and resuspended pollen grain solution. The oil, PEI/RNP solution , and sonicated pollen grains suspension were each loaded in a syringe tipped PrecisionGlide^{™} needle (BDbiosciences) and then the syringes were loaded onto SPC/ZU-I independent syringe pumps and controlled for a constant flow rate 1.5µL/ for the oil mixture while the flow rate for the pollen and the RNP/PEI suspensions were varied between 0.7 - 1.2 µl/min to obtain desired droplet size between 70-100µM respectively, using a digital Controller (SHE-SPM/ZU-I-3 flow controller). The droplets were trapped in the reservoir of the chip for imaging and additional droplets were collected in a vial on ice. The droplets containing both RNP and pollen grain are then analyzed by microscopy imaging and sorted by FACS based on the fluorescence (Figure 5).

### Female flower fertilization

The encapsulated pollen grains were suspended 500uL of germination medium (10% sucrose, 0.005% H₃BO₃, 10 mM CaCl2, 0.05 mM KH₂PO₄, 6% PEG 4000) and was directly applied on mature female flower for fertilization and the flowers were immediately covered with plastic bags.

### Example 2 : Gene edition of PDS1 gene in hemp pollen grain

### Plasmid construction

Guides targeting PDS1 coding sequence were designed according to the gene sequences available under the gene ID 115722384, targeting exon 5 with the following sequences including 4 base pairs adaptors for golden gate cloning.
Guide 1: AGGGAGCTGGCAGAGCATCG (SEQ ID NO: 1)
Guide 2: CAATGCCCAGCAAGCCAGGA (SEQ ID NO: 2)

Both guides were cloned into a level 1 plasmid and assembled into a level 2 plasmids containing attL1 and attL2 recombination site, downstream crRNA sequence, each of them under the control of U6 promoter. To optimize the CRISPR system, self-cleaving ribozyme were added to facilitate accurate 3'-end processing of the crRNA transcript. Finally, the entire cassette was cloned into an expression vector containing the appropriate nuclease fused in N-ter with a GFP, LB and RB border for genome integration and a kanamycin resistance gene.

### Plant material and pollen sampling

Pollen grains were collected from Felina32 male flowers of hemp (*Cannabis sativa*). Hemp seeds were sown in a soil mix of John Innes One, John Innes Two, Vermiculite, Perlite (1:1:1:1) into pots of 9x9x10cm and grown in a growth room under 24 hour blue/ red light. Fresh pollen grains were collected from mature anthers by shaking male flowers.

### Pollen resuspension and aperture opening

10 mg of pollen grains were placed into a 5mL Eppendorf tube and resuspended in 4 mL of resuspension buffer and incubated for 30 minutes on ice. Pollen grains suspension was then treated with an ultrasonic homogenizer, on ice, by submerging the probe of the homogenizer into the pollen grains suspension, with 30% amplitude, 120W for 1 second each time with an interval of 3 second for 90 sec effective time. The suspension was then incubated on ice for 30 minutes and centrifuge 5 min, 3500g to remove the supernatant.

### Pollen transformation & Microfluidic microdroplets generator

PEI stock solution was prepared to a final concentration of 1 mg/ml in a buffer containing 25mM MES pH6. The appropriate quantity of plasmid was mixed to 160 µg of PEI to obtain a final ratio PEI:DNA 4/1 and added to the pollen grain. The obtained suspension was then used to be loaded in the microfluidic device.

### Female flower fertilization & seed collection

The encapsulated pollen grains were suspended 500uL of germination medium (10% sucrose, 0.005% H₃BO₃, 10 mM CaCl2, 0.05 mM KH₂PO₄, 6% PEG 4000) and was directly applied on mature female flower for fertilization and the flowers were immediately covered with plastic bags. The resulting seeds were collected from each individual inflorescence.

### CRISPR mutant screening

Seeds were surface sterilized in 1% H₂O₂ for 72 hours and sown on MS1/4 with 100 ug/mL of kanamycin. Leaf discs from albino plants (corresponding to the expected PDS mutants) were sampled and flash frozen in liquid nitrogen for DNA extraction. DNA extraction was performed according to *Garcia et al., 2016* protocol. PCR amplification of the targeted area was performed using the following primers:
Primer forward: CGGTGACTGGTATGAGACCGG (SEQ ID NO: 3)
Primer reverse: CAGGTCAGCATCTCATTGTTCCG (SEQ ID NO: 4)

Positive plants were then transferred in soil and grown in the growth chamber until maturity.

### REFERENCES

- Abdollahi, M. R., et al. "An efficient method for transformation of pre-androgenic, isolated brassica napus microspores involving microprojectile bombardment and agrobacterium-mediated transformation." Acta Physiologiae Plantarum, vol. 31, no. 6, 2009, pp. 1313-1317, https://doi.org/10.1007/s11738-009-0365-5.
- Abdul-Baki, Aref A., et al. "DNA uptake during electroporation of germinating pollen grains." Plant Science, vol. 70, no. 2, 1990, pp. 181-190, https://doi.org/10.1016/0168-9452(90)90132-8.
- Brew-Appiah, Rhoda A., et al. "Generation of doubled haploid transgenic wheat lines by microspore transformation." PLoS ONE, vol. 8, no. 11, 2013, https://doi.org/10.1371/journal.pone.0080155.
- Demirer, Gozde S., et al. "Carbon nanotube-mediated DNA delivery without transgene integration in intact plants." Nature Protocols, vol. 14, no. 10, 2019, pp. 2954-2971, https://doi.org/10.1038/s41596-019-0208-9.
- Hess, Dieter. "Investigations on the intra- and interspecific transfer of anthocyanin genes using pollen as vectors." Zeitschrift Für Pflanzenphysiologie, vol. 98, no. 4, 1980, pp. 321-337, https://doi.org/10.1016/s0044-328x(80)80262-5.
- Garcia, Virginie, et al. "Rapid identification of causal mutations in tomato EMS populations via mapping-by-sequencing." Nature Protocols, vol. 11, no. 12, 2016, pp. 2401-2418, https://doi.org/10.1038/nprot.2016.143.
- Kwak, Seon-Yeong, et al. "Chloroplast-selective gene delivery and expression in planta using chitosan-complexed single-walled carbon nanotube carriers." Nature Nanotechnology, vol. 14, no. 5, 2019, pp. 447-455, https://doi.org/10.1038/s41565-019-0375-4.
- Lew, Tedrick Thomas, et al. "Nanocarriers for transgene expression in pollen as a plant biotechnology tool." ACS Materials Letters, vol. 2, no. 9, 2020, pp. 1057-1066, https://doi.org/10.1021/acsmaterialslett.0c00247.
- Lv, Zongyou, et al. "Nanoparticle-mediated gene transformation strategies for plant genetic engineering." The Plant Journal, vol. 104, no. 4, 2020, pp. 880-891, https://doi.org/10.1111/tpj. 14973.
- Masud, Mostafa Kamal, et al. "Superparamagnetic nanoarchitectures for disease-specific biomarker detection." Chemical Society Reviews, vol. 48, no. 24, 2019, pp. 5717-5751, https://doi.org/10.1039/c9cs00174c.
- Matthews, BenjaminF., et al. "Expression of a foreign gene in electroporated pollen grains of tobacco." Sexual Plant Reproduction, vol. 3, no. 3, 1990, https://doi.org/10.1007/bf00205223.
- Negrutiu, I., et al. "Hybrid genes in the analysis of transformation conditions." Plant Molecular Biology, vol. 8, no. 5, 1987, pp. 363-373, https://doi.org/10.1007/bf00015814.
- Serag, Maged F., et al. "Introducing carbon nanotubes into living walled plant cells through cellulase-induced nanoholes." RSC Adv., vol. 2, no. 2, 2012, pp. 398-400, https://doi.org/10.1039/cira00760b.
- Stöger, Eva, et al. "Comparison of different techniques for gene transfer into mature and immature tobacco pollen." Transgenic Research, vol. 1, no. 2, 1992, pp. 71-78, https://doi.org/10.1007/bf02513024.
- Twell, David, et al. "Transient expression of chimeric genes delivered into pollen by microprojectile bombardment." Plant Physiology, vol. 91, no. 4, 1989, pp. 1270-1274, https://doi.org/10.1104/pp.91.4.1270.
- Van Wert, Sally L., and James A. Saunders. "Reduction of nuclease activity released from germinating pollen under conditions used for pollen electrotransformation." Plant Science, vol. 84, no. 1, 1992, pp. 11-16, https://doi.org/10.1016/0168-9452(92)90202-w.
- Wang, Zuo-Ping, et al. "Efficient and genotype independent maize transformation using pollen transfected by dna-coated magnetic nanoparticles." Journal of Integrative Plant Biology, vol. 64, no. 6, 2022, pp. 1145-1156, https://doi.org/10.1111/jipb. 13263.
- Wong, Min Hao, et al. "Lipid exchange envelope penetration (LEEP) of nanoparticles for Plant Engineering: A universal localization mechanism." Nano Letters, vol. 16, no. 2, 2016, pp. 1161-1172, https://doi.org/10.1021/acs.nanolett.5b04467.
- Yang, Liyan, et al. "Expression of foreign genes demonstrates the effectiveness of pollen-mediated transformation in Zea Mays." Frontiers in Plant Science, vol. 8, 2017, https://doi.org/10.3389/fpls.2017.00383.
- Zhao, Xiang, et al. "Pollen magnetofection for genetic modification with magnetic nanoparticles as gene carriers." Nature Plants, vol. 3, no. 12, 2017, pp. 956-964, https://doi.org/10.1038/s41477-017-0063-z.

## Claims

1. Method of transforming pollen grains, the method comprising the steps of:
(a) providing pollen grains having at least one pore,
(b) providing a genome editing composition, the composition comprising a ribonucleoprotein (RNP) complex comprising (i) a RNA-guided endonuclease or a nucleic acid comprising a nucleotide sequence encoding the RNA-guided endonuclease and (ii) a guide RNA (gRNA) or a nucleic acid comprising a nucleotide sequence encoding the gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby obtaining transformed pollen grains.

2. Method of genome editing pollen grains, the method comprising the steps of:
(a) providing pollen grains having at least one pore,
(b) providing a genome editing composition, the composition comprising a ribonucleoprotein (RNP) complex comprising (i) a RNA-guided endonuclease or a nucleic acid comprising a nucleotide sequence encoding the RNA-guided endonuclease and (ii) a guide RNA (gRNA) or a nucleic acid comprising a nucleotide sequence encoding the gRNA,
(c) subjecting the pollen grains to sonication thereby obtaining sonicated pollen grains,
(d) incubating the sonicated pollen grains and the genome editing composition into microdroplets, and
(e) allowing penetration of the RNP complex into the sonicated pollen grains thereby generating genome edited pollen grains.

3. The method according to any of the preceding claims, wherein the pollen grains are collected from a plant of the poaceae family, such as a plant of the genus oryza or avena.

4. The method according to any of the preceding claims, wherein the pollen grains are in a solution.

5. The method according to any of the preceding claims, wherein the sonication step opens the at least one pore.

6. The method according to any of the preceding claims, wherein, the sonication step separates the pollen grains that are stuck together.

7. The method according to any of the preceding claims, wherein the sonicated pollen grains and the genome editing composition are introduced into the microdroplets using a microfluidic microdroplets generator.

8. The method according to any of the preceding claims, wherein the RNP complex is labelled with a luminescent dye, such as a fluorescent dye.

9. The method according to any of the preceding claims, wherein the microdroplets are lipid microdroplets.

10. The method according to any of the preceding claims, wherein the RNA-guided endonuclease is selected from MAD7, CAS9, CAS12, Cpf 1, C2c1, C2c2.

11. The method according to any of claims of the preceding claims, wherein the gRNA comprises a crRNA-tracrRNA duplex or a crRNA-tracrRNA chimera.

12. The method according to any of the preceding claims, wherein the composition comprising a genome editing composition also comprises a nonviral nucleotide delivery reagent, such as polyethylenimine (PEI).

13. The method according to any of the preceding claims, wherein the genome editing composition also comprises a donor nucleic acid.

14. The method according to any of the preceding claims, further comprising a step of screening the transformed pollen grains after step (e).

15. A method of obtaining a gene edited homozygous plant, the method comprising the step of pollinating a plant ovule with a pollen grain obtained by the method according to any of claims 1 to 14 thereby obtaining a genome edited homozygous plant, and optionally further comprising a step of screening the genome edited plants.
